Europäisches Patentamt

European Patent Office

Office européen des brevets

(19)

(11) Publication number: **0 033 212**
A2

(12) # EUROPEAN PATENT APPLICATION

(21) Application number: **81300240.9**

(22) Date of filing: **20.01.81**

(51) Int. Cl.³: **C 07 C 29/15**
C 07 C 27/06, C 07 C 31/08
C 07 C 47/06, C 07 C 53/08
C 07 C 45/49

(30) Priority: **24.01.80 GB 8002466**
**24.01.80 GB 8002467**

(43) Date of publication of application:
**05.08.81 Bulletin 81/31**

(84) Designated Contracting States:
**BE DE FR GB IT NL**

(71) Applicant: **The British Petroleum Company Limited**
**Britannic House Moor Lane**
**London EC2Y 9BU(GB)**

(72) Inventor: **Ball, William John**
**The British Petroleum Company Ltd. Chertsey Road**
**Sunbury-on-Thames Middlesex TW16 7LN(GB)**

(72) Inventor: **Stewart, David Gordon**
**The British Petroleum Company Ltd. Chertsey Road**
**Sunbury-on-Thames Middlesex TW16 7LN(GB)**

(74) Representative: **Harry, John et al,**
**BP INTERNATIONAL LIMITED Patents and Licensing**
**Division Chertsey Road**
**Sunbury-on-Thames Middlesex TW16 7LN(GB)**

(54) Process for the production of oxygenated hydrocarbons from synthesis gas.

(57) Oxygenated hydrocarbons are produced by reacting in a liquid medium carbon monoxide and hydrogen in the presence of a particulate catalyst comprising a supported rhodium component at an elevated temperature, typically in the range 150 to 300°C and an elevated pressure, typically greater than 50 bars. Suitable liquid media include water, an alcohol, a carboxylic acid, an ester, a ketone, an ether or an aromatic hydrocarbon. The rhodium catalyst may be supported on silica and is desirably combined with other metals.

EP 0 033 212 A2

Croydon Printing Company Ltd.

1

## PROCESS FOR THE PRODUCTION OF OXYGENATED HYDROCARBONS FROM SYNTHESIS GAS.

The present invention relates generally to a process for the production of oxygenated hydrocarbon compounds such as acetic acid, ethanol and/or acetaldehyde. In particular the invention concerns the production of such oxygenated compounds by reacting a mixture of carbon monoxide and hydrogen, hereinafter to be referred to as synthesis gas, with a catalyst comprising a supported rhodium component in a liquid medium.

Oxygenated hydrocarbons such as acetic acid, ethanol and acetaldehyde are valuable industrial products. On a commercial scale acetic acid is generally produced either by oxidation of paraffinic hydrocarbon fractions or by carbonylation of methanol; ethanol is produced either by fermentation of natural products, e.g. molasses, or by hydration of ethylene in the presence of an acid catalyst and acetaldehyde is produced by the oxidation of ethanol or by direct oxidation of ethylene as in the Wacker process. The dwindling reserves of crude oil from which many of the above feedstocks are derived and the associated need to utilise fully the remaining natural resources such as coal and the vast amounts of gases, e.g. methane potentially available from the exploitation of North Sea oilfields, has stimulated research into the utilisation of synthesis gas which can readily be obtained not only from crude oil but also from both coal and methane gas. In this context attention has been directed to two different approaches, firstly to the gas phase hydrogenation of carbon monoxide in the presence of heterogeneous catalysts and secondly to the liquid phase homologation of methanol, itself obtainable from synthesis gas, in the presence of soluble cobalt catalysts.

Much of the early work on the hydrogenation of carbon monoxide involved the use as catalysts of the iron group metals, ruthenium and various metal oxide systems. One general disadvantage of such systems is that catalysts which possess acceptable activity generally tend to be unselective, i.e. they produce a wide spectrum of products including both hydrocarbons and oxygenated hydrocarbons having a broad distribution of carbon numbers. This not only complicates the recovery of the desired products but also results in wastage of reactants to undesirable products. On the other hand those catalysts having acceptable selectivity generally have a low activity thereby necessitating recycle of large quantities of unchanged reactants. In US Application Serial No 541,661 (Union Carbide Corp) published as a priority document for Netherlands application No 7500916 there is disclosed a process which, it is claimed, overcomes the aforesaid disadvantages of the prior art processes. The process for selectively producing $C_2$ – oxygenated hydrocarbons involves continuously contacting synthesis gas with a heterogeneous catalyst essentially comprising rhodium metal under reaction conditions correlated so as to favour the formation of a substantial proportion of acetic acid, ethanol and/or acetaldehyde. Subsequent patent applications describe the production of ethanol by contacting synthesis gas with a rhodium/iron catalyst (US Serial No 541,660 published as a priority document for Netherlands application No 7500918), a rhodium/manganese catalyst (GB 1549437), a rhodium/ molybdenum or rhodium/tungsten catalyst (USP 4096164), a rhodium/ ruthenium catalyst (USP 4101450) and a rhodium/uranium/thorium catalyst (USP 4162262). Recently published European applications EP 4-653 and 4-656 (Hoechst) disclose the use of a supported rhodium/ magnesium/halide ion catalyst. Our copending European application publication No. 0018763 describes the use of a supported rhodium/ chromium catalyst for the production of $C_1$ to $C_4$ oxygenated hydrocarbons from synthesis gas.

Optimally for the heterogeneous hydrogenation of carbon monoxide reaction temperatures are in the range 250 to 300°C and reaction pressures are in the range 20 to 300 bar.

The liquid phase homologation of methanol to ethanol has been known for many years. Thus in a paper published in Science $\underline{113}$, 206 (1951), Wender, Friedel and Orchin reported that methanol was reacted with synthesis gas ($1H_2$:1CO) in the presence of dicobalt octacarbonyl as catalyst to produce methyl formate (2%), methyl acetate (9.0%), ethyl alcohol (38.8%), ethyl acetate (6.3%), propyl alcohol (4.7%), butyl alcohol (0.9%), methane (8.5%), propyl acetate (0.1%) and a small amount of unidentified product, the total conversion of methanol being 76.4%. Since that time many attempts to improve the yield and selectivity to ethanol have been reported. A common feature of the more recently reported work has been the addition to the cobalt catalyst of promoters such as iodides or bromides and/or organo-phosphorus compounds, e.g. phosphines. Whilst the addition of promoters has resulted in some improvement in yield and selectivity further improvements have resulted from the addition to the initial reaction mixture of various additives as described in our Belgian Patent No: 867548 and the published specifications of our European applications Nos. 78300608.3 and 79300174.4. Optimally the process is operated at a temperature below 200°C and a pressure in the range 50 to 200 bar.

We have now found the surprisingly the supported rhodium catalysts conventionally employed in the gaseous phase hydrogenation of carbon monoxide are also effective catalysts for the production of oxygenated hydrocarbon compounds in liquid media. Under these conditions the heat liberated from such exothermic reactions is more readily dispersed. The reaction can be operated under the milder reaction conditions encountered in liquid phase reactions and the catalyst separation difficulties commonly encountered in homogeneous liquid phase reactions can be avoided because the catalyst, although effective in the liquid medium, is nevertheless heterogeneous.

Accordingly the present invention provides a process for the production of oxygenated hydrocarbon compounds from carbon monoxide and hydrogen which process comprises reacting, in a liquid medium and under conditions of elevated temperature and pressure, carbon monoxide and hydrogen in the presence of a particulate catalyst comprising a supported rhodium component.

Mixtures of the gases hydrogen and carbon monoxide are abundantly available in the form of synthesis gas. Methods for preparing synthesis gas are well-known in the art and usually involve the partial oxidation of a carbonaceous substance, e.g. coal. Alternatively synthesis gas may be prepared, for example, by the catalytic steam reforming of methane. Although it is preferred to use substantially pure synthesis gas the presence of such impurities as carbon dioxide and nitrogen can be tolerated. On the other hand impurities which have a deleterious effect on the reaction should be avoided. The ratio of hydrogen to carbon monoxide in the synthesis gas may vary widely. Normally the molar ratio of hydrogen to carbon monoxide may be in the range from 20:1 to 1:20, preferably from 5:1 to 1:5 and even more preferably from 1:1 to 2:1. Methods for adjusting the molar ratio by the so-called 'shift reaction' are well-known to those versed in the art.

The catalyst comprises a supported rhodium component. A wide variety of support materials may be employed. Suitable support materials include silica, alumina, silica/alumina, magnesia, thoria, titania, chromia, zirconia and active carbon, of which silica is preferred. Zeolite molecular sieves and in particular crystalline aluminosilicates may also be employed. Suitably the support has a relatively high surface area. The support may have a surface area up to 350 square metres per gram (BET low temperature nitrogen absorption isotherm method), preferably in the range 1 to 300 square metres per gram. Whilst the actual form of the rhodium component under the reaction conditions is not known with any degree of certainty it is probable that it is in the metallic form under the reducing conditions prevailing. Thus the rhodium component may be added to the support in the form of the metal itself or in the form of a metal compound. The rhodium component may be deposited on the support by any of the techniques commonly used for catalyst preparation. Although it is possible to add particles of the metal to the support it is preferred to use the techniques of impregnation from an organic or inorganic solution, precipitation, coprecipitation or cation exchange. Conveniently the catalyst may be prepared by impregnating the support with a solution of an inorganic or organic rhodium compound. Suitable compounds are the salts of the metal e.g. the halide, particularly the chloride and nitrate. Following impregnation the catalyst is preferably dried and calcined. The amount of the rhodium component on the support may suitably be in the range of from 0.01 to 25 weight percent, preferably from 0.1 to 10 weight percent, of the metal based on the combined weight of the metal and the support. One or more additional metal components may also be incorporated in the catalyst. Suitable metals include magnesium, iron, zirconium, manganese, molybdenum, tungsten, silver, rhenium, ruthenium, chromium, uranium and thorium, suitably in an amount in the range from 0.1 to 10 weight percent based on the combined weight of the metals and the support.

The support may also be activated by the addition of a suitable metal or non-metal component prior to incorporation of the rhodium component. Whilst a wide variety of such metals and non-metals may be added, the alkali metals, magnesium, thorium, manganese, rhodium, iron, chromium, molybdenum, boron and phosphorus are specific examples of such materials. Any of the known techniques for catalyst preparation hereinafter referred to may be used for addition of the activating material. In the case of a metal activator the support is preferably impregnated with a solution of a compound of the metal, suitably the nitrate or chloride, and is thereafter dried, suitably by evaporation and calcined. The activated support is then in a suitable condition for addition of the rhodium component and optionally other metal compounds. The amount of activator added may suitably be in the range 0.01 to 50 weight percent, preferably from 1 to 25 weight percent, of the activator element based on the combined weight of the activator element and the support.

The catalyst is employed in particulate form. Suitably the particle size may be in the range 66 to 500 microns, preferably in the range 150 to 300 microns.

The liqiud medium may suitably be an inorganic liquid or an organic liquid. Suitably the inorganic liquid may be water or an aqueous solution of a mineral acid or salt and is preferably water. The organic liquid may suitably be an alcohol, a carboxylic acid, an ester, a ketone or an ether. Of the alcohols methanol and ethanol are preferred. An example of a suitable carboxylic acid is acetic acid. Examples of suitable esters are methyl acetate, ethyl acetate, butyl acetate and ethyl propionate. An example of a suitable ketone is cyclohexanone. An example of a suitable ether is diphenyl ether. Using methanol or methyl esters as the organic liquid it may well be that part of the methanol or methyl ester reacts with synthesis gas and is thereby converted to oxygenated hydrocarbons. Alternatively the organic liquid medium may suitably be an aromatic or a paraffinic hydrocarbon. Examples of suitable aromatic hydrocarbons include benzene, toluene and xylene. An example of a suitable paraffinic hydrocarbon is heptane.

The amount of catalyst employed may suitably be between 1 and 20% by weight of the liqiud.

Conditions of elevated temperature and pressure which may suitably be employed are a temperature in the range 150 to 300°C, preferably 180 to 250°C and a pressure greater than 50 bars, preferably in the range 100 to 300 bars.

In addition to the catalyst a co-catalyst may be employed. Suitably the co-catalyst may be a soluble compound, e.g. a halide, of one or more other metals of Group VIII of the Periodic Table. Suitable metals of Group VIII include ruthenium, osmium, platinum, palladium, iridium and rhodium. The metal may be added, for example, in the form of a carbonyl, e.g. $Ru_3(CO)_{12}$, a beta-diketonate, e.g. $Ru(acetoacetonate)_3$, a halide, e.g. $RuCl_3 \cdot 3H_2O$ or a salt of a carboxylic acid, e.g. ruthenium acetate.

The process of the present invention may be carried out batchwise or continuously, preferably continuously.

The batch reaction may be carried out by charging the catalyst and the liquid to an autoclave equipped with a stirrer, pressurising to the reaction pressure with carbon monoxide and hydrogen and thereafter heating the autoclave to the reaction temperature. The initial reaction pressure may be restored by periodic injection of further carbon monoxide and hydrogen. The residence time for batch operation, which is defined as the length of time for which the reactor is at the reaction temperature, may suitably be up to 8 hours, but is preferably in the range from 10 to 180 minutes. The continuous process may suitably be carried out by passing the carbon monoxide, hydrogen and liquid through a bed of the catalyst packed in a tubular reactor. The residence time for continuous operation, which is defined as:-

$$\frac{\text{Volume of the reactor occupied by solid catalyst}}{\text{Total flow of gas into the reactor (litres/hour at STP)}}$$

may suitably be up to 8 hours, preferably in the range from 10 to 180 minutes.

The invention will now be illustrated by reference to the following Examples.

Example 1

Catalyst Preparation

Ammonium heptamolybdate tetrahydrate (0.42g), chromium nitrate nonahydrate (3.8g) and rhodium trichloride trihydrate (1.3g) were dissolved in deionised water (20ml) and the resulting solution was added to Davison silica, grade 59 (10g, 8-16 mesh B.S.S.), which had been dried at 120°C for 4 days. The mixture was evaporated to dryness on a steam-bath and then dried at 120°C for 16 hours. The dry catalyst was crushed to pass >30 mesh and then reduced in a current of hydrogen at 450°C for 6 hours before it was tested.

Catalyst Testing

A stainless steel, magnetically - stirred autoclave equipped for pressurised reactions was charged with methanol (250ml) and the Cr/Mo/Rh/SiO$_2$ catalyst (12g) prepared as described under

'Catalyst Preparation'. The system was purged with hydrogen and then heated to 230°C. The autoclave was then pressurised to 150 bars with a mixture of carbon monoxide and hydrogen (1:1 molar). During the course of the reaction, whenever the pressure in the autoclave dropped to 140 bars a fresh charge of carbon monoxide and hydrogen (1:1 molar mixture) was added thereby increasing the reaction pressure to 150 bars. After three hours at 230°C the autoclave was allowed to cool and the reaction product was fully analysed. The product was shown to contain ethanol (2.1%), methyl acetate (0.7%) and n-propanol (1.0% wt/wt). The productivity for ethanol was 7g/hr/kg methanol.

Example 2

Catalyst preparation

Zirconium tetrachloride (1.3g), ammonium heptamolybdate tetrahydrate (0.42g), chromium nitrate nonahydrate (3.8g) and rhodium trichloride trihydrate (1.3g) were dissolved in deionised water (30 ml) and the resulting solution was added to Davison silica, grade 59 (10 g, 8-16 mesh B.S.S.). The mixture was evaporated to dryness on a steam-bath and dried at 120°C for 16 hours. The dry catalyst was crushed to pass >30 mesh and then reduced in a current of hydrogen at 450°C for three hours before it was tested.

Catalyst Testing

A stainless steel, magnetically - stirred autoclave equipped for pressurised reactions was charged with n-heptane (250 ml) and the $Cr/Mo/Rh/Zr/SiO_2$ catalyst (24 g) prepared as described under 'Catalyst Preparation'. The system was purged with hydrogen and then heated to 230°C. The autoclave was then pressurised to 150 bars with a mixture of carbon monoxide and hydrogen (1:1 molar). During the course of the reaction, whenever the pressure in the autoclave dropped to 140 bars a fresh charge of carbon monoxide and hydrogen (1:1 molar mixture) was added, thereby increasing the reaction pressure to 150 bars. After three hours at 230°C the autoclave was allowed to cool and the reaction product was fully analysed. The product was shown to contain acetaldehyde (1.3%), propionaldehyde (0.2%), methanol (0.3%) methyl acetate (0.1%), ethanol (0.7%), ethyl acetate (0.5%), n-propanol (0.1%) and n-propyl acetate (0.06% wt/wt). The productivity was 8.3 g of $C_2$ - compounds/hr/kg n-heptane.

Example 3

Catalyst Preparation

Zirconium tetrachloride (10.4 g), chromium nitrate nonahydrate (30.4 g) and rhodium trichloride trihydrate (10.4 g) was dissolved in deionised water (125 ml) and the resulting solution was added to Davison silica grade 59 (80 g, >30 mesh B.S.S.). The mixture was evaporated to dryness on a steam-bath and dried at 120°C for 16 hours.

Ammonium heptamolybdate tetrahydrate (3.36 g) was dissolved in deionised water (125 ml) and this solution was added to the impregnated silica prepared as above. The mixture was evaporated to dryness on a steam bath and dried at 120°C for 16 hours. The dry catalyst was then reduced in a current of hydrogen at 450°C for 16 hours before it was tested.

Catalyst Testing

A stainless steel, magnetically-stirred autoclave equipped for pressurised reactions was charged with deionised water (247.3g) and the $Cr/Rh/Zr/Mo/SiO_2$ catalyst (22.9 g) prepared as described under 'Catalyst Preparation'. The system was purged with hydrogen and then heated to 230°C. The autoclave was then pressurised to 150 bars with a mixture of carbon monoxide and hydrogen (1.1. molar). During the course of the reaction, whenever the pressure in the autoclave dropped to 140 bars a fresh charge of carbon monoxide and hydrogen (1:1 molar mixture) was added, thereby increasing the reaction pressure to 150 bars. After 3 hours at 230°C the autoclave was allowed to cool and the reaction product was fully analysed. The product was shown to contain methanol (1.4%), ethanol (0.85%) and n-propanol (0.16% wt/wt).

## Claims

1.  A process for the production of oxygenated hydrocarbon compounds from carbon monoxide and hydrogen which process comprises reacting, in a liquid medium and under conditions of elevated temperature and pressure, carbon monoxide and hydrogen in the presence of a particulate catalyst comprising a supported rhodium component.

2.  A process according to claim 1 wherein the rhodium component is supported on silica.

3.  A process according to either one of claims 1 or 2 wherein the amount of the rhodium component on the support is in the range from 0.1 to 10 weight percent of the metal based on the combined weight of the metal and the support.

4.  A process according to any one of claims 1 to 3 wherein in addition to the rhodium component there is also incorporated in the catalyst one or more of the metals magnesium, iron, zirconium, manganese, molybdenum, tungsten, silver, rhenium, ruthenium, chromium, uranium and thorium.

5.  A process according to any one of claims 1 to 4 wherein the liquid medium is an alcohol, a carboxylic acid, an ester, a ketone or an ether.

6.  A process according to claim 5 wherein the liquid medium is methanol, ethanol, acetic acid, methyl acetate, ethyl acetate, butyl acetate, ethyl propionate, cyclohexanone or diphenyl ether.

7.  A process according to any one of claims 1 to 4 wherein the liquid medium is an aromatic or a paraffinic hydrocarbon.

8.  A process according to claim 7 wherein the hydrocarbon is benzene, toluene, xylene or heptane.

9.  A process according to any one of claims 1 to 4 wherein the liquid medium is water.

10. A process according to any one of claims 1 to 9 wherein the conditions of elevated temperature and pressure are a temperature in the range 150 to 300°C and a pressure greater than 50 bars.

11. A process according to claim 10 wherein the temperature is in the range 180 to 250°C and the pressure is in the range 100 to 300 bars.

12. A process according to any one of claims 1 to 11 wherein there is employed as a co-catalyst a soluble compound of one or more other metals of Group VIII of the Periodic Table.

13. A process according to claim 12 wherein the metal of Group VIII is ruthenium, osmium, platinum, palladium, iridium or rhodium.

14. A process according to any one of claims 1 to 13 wherein the process is carried out continuously.